# EUROPEAN PATENT SPECIFICATION

(11) **EP 0 760 232 B1**
(45) Date of publication and mention of the grant of the patent: **21.05.2003**
(21) Application number: 96305939.9
(22) Date of filing: 14.08.1996
(51) Int. Cl.: A61F 2/16

(54) **Intraocular lens**
Intraokulare Linse
Lentille intra-oculaire

(30) Priority: 31.08.1995 JP 24862395; 29.09.1995 JP 27632595
(43) Date of publication of application: 05.03.1997
(73) Proprietor: NIDEK CO., LTD, Gamagori-shi, Aichi 443-0035 (JP)
(72) Inventor: Sunada, Tsutomu, Aichi-ken, 441-3101 (JP)
(74) Representative: Blatchford, William Michael

(56) References cited:
- EP-A- 0 246 216
- EP-A- 0 346 245
- FR-A- 2 700 466
- GB-A- 2 180 160
- US-A- 4 624 670
- US-A- 5 374 663

## Description

The present invention relates to an intraocular lens, and more particularly to an intraocular lens inserted into each eye after crystalline lenses have been removed therefrom.

An intraocular lens has been known which is inserted into each eye after crystalline lenses have been taken out to treat a cataract. In general, the present main-current intraocular lens has an optical portion corresponding to a lens body and loop-shaped support portions for supporting the optical portion. In recent years, a so-called single-piece type intraocular lens is widespread wherein an optical portion and support portions have been shaped in integral form.

The thickness of an edge portion of the optical portion employed in the single-piece type intraocular lens has heretofore depended on the thickness of each support portion so that the support portions are continuously joined to the optical portion. That is, the edge portion has been fabricated under the same thickness as that of each support portion.

The degree of an intraocular lens used as an alternative to each of crystalline lenses normally becomes much higher and the central portion of the lens that constitute an optical portion, becomes so thicker than that of an edge portion thereof. Accordingly, the thickness of the edge portion may generally be identical to that of each of support portions.

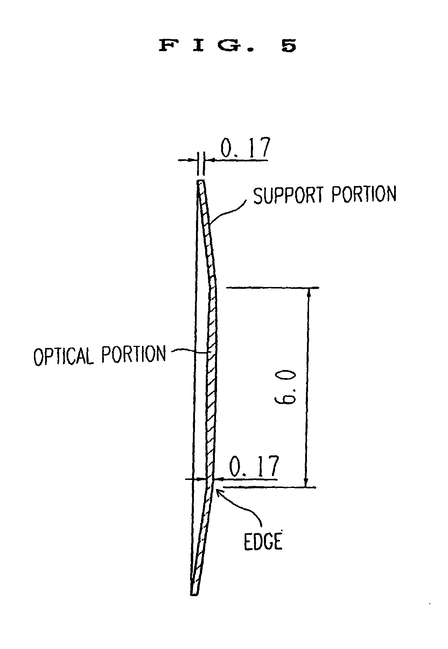

However, lenses whose degrees are relatively low, are often required among those whose eyes have been operated. Since the central portion of each lens whose degree is low, is not so thick as shown in Fig. 5 (which is a cross-sectional view showing the state in which a lens material has been subjected to a process up to a cutting process) under the thickness of an edge portion, which is determined depending on the thickness of each of support portions, the lens whose degree is low, still has a problem in terms of durability.

A method of increasing the entire thickness of each of support portions and further increasing the central portion of a lens whose degree is low, is considered as a method of sufficiently ensuring the thickness of the central portion of the lens. Since, however, characteristics such as a flection of the support portions are impaired in this case, a problem arises in terms of its usage.

Further, an intraocular lens added with a coloring agent or an ultraviolet-ray absorbent agent has a problem that light transmittance changes with a change in the thickness of an optical portion and hence the level or degree capable of correcting chromatopsia will vary.

An intraocular lens according to the preamble of claim 1 is known from document US-A-5 374 663.

The present invention has been made in view of the above circumstances and has an object to overcome the above problems and to provide, as its technical issue, an intraocular lens capable of sufficiently ensuring the thickness of an optical portion without impairing characteristics of support portions as compared with a lens whose degree is low.

It is another object of the present invention to provide an intraocular lens containing pigment, of a type wherein light transmittance does not greatly change even if the degree of the lens changes.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

In accordance with the present invention, there is provided an intraocular lens according to claim 1.

In one embodiment, the intraocular lens of this invention comprises an optical portion having a refractive power, support portions for supporting the optical portion, the support portions being formed integrally with the optical portion, and a thickness transition portion whose thickness gradually increases toward an edge portion of the optical portion.

In another embodiment of the present invention, the intraocular lens comprises an optical portion having a refractive power, support portions for supporting the optical portion, the support portions being formed integrally with the optical portion, and a thickness transition portion whose thickness gradually increases toward the optical center of the optical portion as compared with a lens whose thickness of optical center and refractive power are identical to those of the intraocular lens within a region excluding a visual field region of an eye to insert the intraocular lens therein.

Also, in another embodiment of the present invention, the intraocular lens comprises an optical portion for containing a coloring agent or an ultraviolet-ray absorbent agent and fixing a thickness of the optical center regardless of refractive power of the lens, support portions for supporting the optical portion, the support portions being formed integrally with the optical portion, and a thickness transition portion for increasing gradually a thickness toward an edge of the optical portion, the thickenss transition portion being provided at the support portions.

Also, another embodiment of the present invention, the intraocular lens comprises an optical portion for containing a coloring agent or an ultraviolet-ray absorbent agent and fixing a thickness of the optical center regardless of refractive power of the lens, support portions for supporting the optical portion, the support portion being formed integrally with the optical portion, and a thickness transition portion for increasing gradually a thickness toward the optical center of the optical portion as compared with a lens whose thickness of optical center and refractive power are identical to those of the intraocular lens within a region excluding a visual field region of an eye to insert the intraocular lens therein.

According to the present invention as has been described above, even a lens having a low degree can ensure a sufficient thickness without impairing the characteristics of support portions as much as possible and narrowing the region of a lens function.

According to the present invention as well, an intraocular lens with a coloring agent contained therein, which corresponds to a change in its degree, can be obtained without impairing the characteristics of support portions while maintaining its transmissivity characteristics.

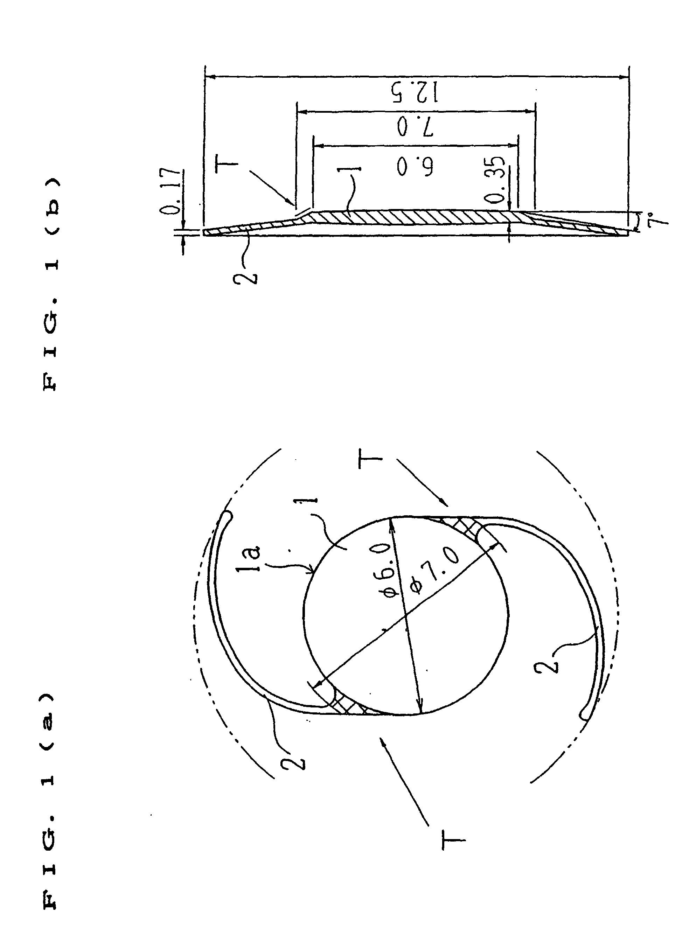

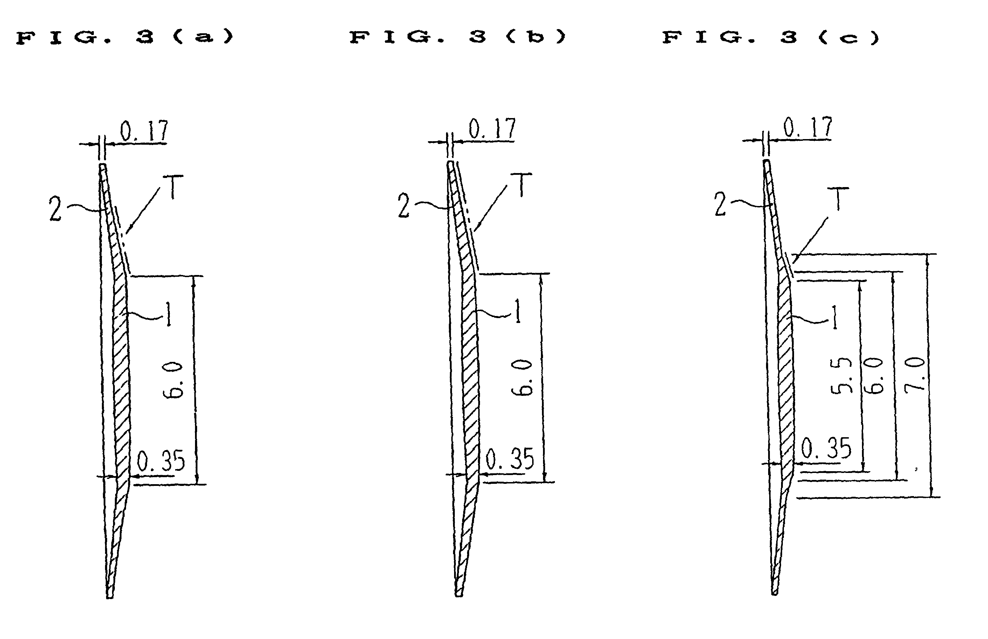

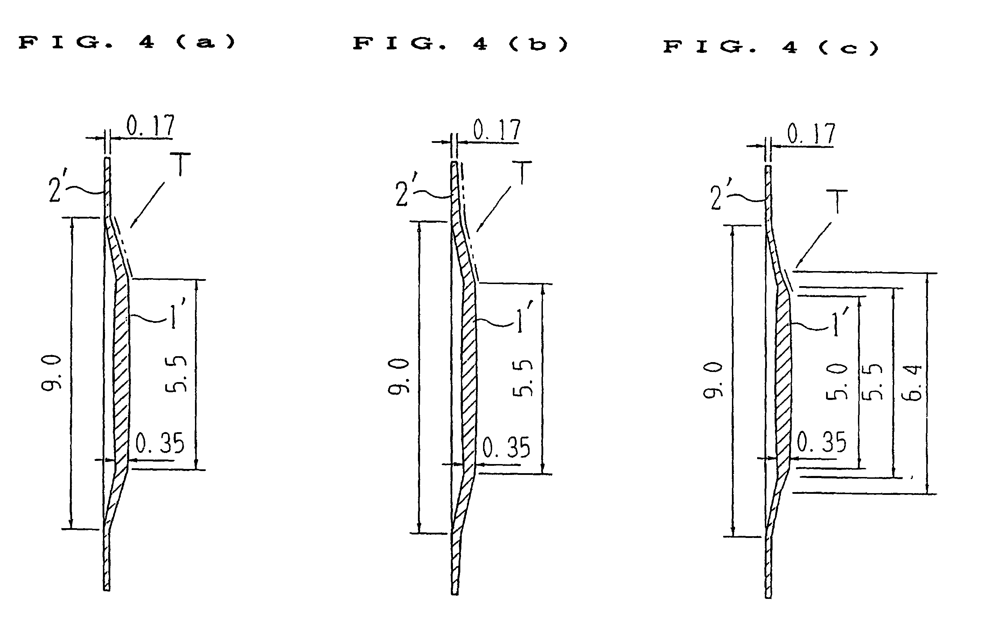

The accompanying drawings, which are incorporated in and constitute a part of this specification illustrate embodiments of the present invention and, together with the description, serve to explain the objects, advantages and principles of the invention. In the drawings:
Figs. 1(a) and 1(b) are plan views showing an intraocular lens according to a first embodiment of the present invention and is a cross-sectional view illustrating the state in which a base material has been subjected to a process up to a cutting process of a lathe to manufacture the intraocular lens.
Figs. 2(a) and 2(b) are views showing an intraocular lens according to a second embodiment of the present invention.
Figs. 3(a) and 3(b) are views illustrating examples of modifications of the intraocular lens of the type according to the first embodiment shown in Fig. 1.
Figs. 4(a) and 4(b) are views showing examples of modifications of the intraocular lens of the type according to the second embodiment shown in Figs. 2(a) and 2(b).
Fig. 5 is a view for describing a conventional intraocular lens having a low degree.
   A detailed description of one preferred embodiment of an intraocular lens embodying the present invention will now be given referring to the accompanying drawings. Fig. 1(a) is a plan view showing an intraocular lens according to a first embodiment of the present invention. Fig. 1(b) is a cross-sectional view illustrating the state in which a base material has been subjected to a process up to a cutting process of a lathe to manufacture the intraocular lens.
   Reference numeral 1 indicates a lens optical portion having a refractive power. Reference numerals 2 indicate two loop-shaped support portions for supporting the lens optical portion 1 in intraocular place. The support portions 2 respectively extend in bent form from the opposed positions on the outer periphery of the lens optical portion 1. The intraocular lens according to the present embodiment is of a single-piece type one in which the lens optical portion 1 and the support portions 2 are integrally formed of a base material of PPMA (Polymethyl metacrylate).
   The diameter of the lens optical portion 1 is 6 mm. The thickness of an edge portion la of the lens optical portion 1 is set to 0.35mm or more. Even if a lens whose refractive power is low, is used, the thickness thereof sufficiently resistant to irradiation of a YAG laser can be ensured at the central portion of the lens.
   The overall length between the respective tip portions of the support portions 2 is 12.5 mm as the distance. The tip portion of each of the support portions 2 is inclined about 7 ° toward the plane orthogonal to the optical axis of the lens. Each of the support portions 2 includes a transition zone T extending from the edge portion la to a region spaced about 0.5 mm away therefrom. The thickness of the transition zone T gradually changes in the radial direction (i.e., changes from 0.35 mm to 0.17 mm). The transition zone T employed in the present embodiment is of a portion so called "junction." Namely, the transition zone T is a portion whose width expands in this region in terms of processing characteristics, and which will not produce deformation such as a flection as a support portion and will not influence its mechanical characteristics. Each of the support portions 2 provided outside the transition zones T has a substantially uniform thickness of 0.17 mm. Thus, each support portion ensures characteristics such as suitable strength and a satisfactory flection.
   After the intraocular lens having such transition zones T has been manufactured by the known processing technique, the mechanical characteristics of the support portions 2 were examined by a compressive test. As a result, no change occurred in characteristic as compared with the conventional lens. It was thus possible to confirm that an increase in thickness of each support portion 2 at the junction whose width has been expanded, would cause no harm to the characteristic of each support portion 2. Further, a problem on processing did not arise either.

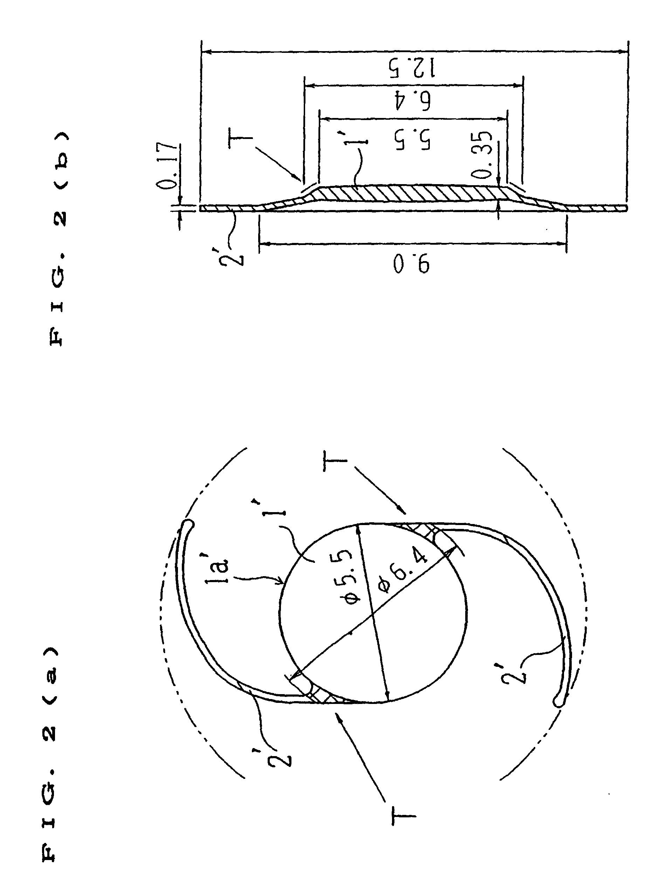

   Figs. 2(a) and 2(b) are views showing an intraocular lens according to a second embodiment of the present invention. The second embodiment is an example of a type different in support-portion configuration from the first embodiment.
   Each of support portions 2' is bend from a point where the diameter thereof is 9 mm. The tip portion of each support portion 2', which extends from the bent point, is vertical to the optical axis of the intraocular lens. The intraocular lens having such a configuration of each support portion provides easy insertion into the lens capsule upon surgical operation and stabilizes fixing to within the lens capsule. Even in the case of the intraocular lens according to the present embodiment, each of transition zones T is provided within a region called "junction" at which the width of each support portion 2' has expanded. The support portion has a substantially uniform thickness of 0.17 mm over a range from the tip portion thereof to a point spaced about 0.45 mm away from the outside of an edge portion 1a'. The thickness of the support portion gradually increases so as to reach 0.35 mm corresponding to the thickness of the edge portion 1a' over the range from the point to an optical portion 1'. Even in the case of the support portions 2' of the intraocular lens, no change occurred in mechanical characteristics thereof.
   Various changes can be made to the intraocular lenses described above. Figs. 3(a) to 3(c) and 4(a) to 4(c) show some modifications of the intraocular lens (individual drawings are illustrated as cross-sectional views each showing the state in which a base material has been subjected to a process up to a cutting process by a lathe).
   Figs. 3(a) to 3(c) illustrate modifications of the intraocular lens of the type showing the first embodiment. Fig. 3(a) shows the modification in which each of transition zones T is formed over a range from an edge portion la to a point spaced about 2 mm away from the outside of the edge portion la. Fig. 3(b) illustrates the modification in which each of transition zones T is formed over a range from an edge portion la to the tip portion of each support portion 2. These provide less changes in mechanical characteristic of each support portion as compared with ones in which the entire thickness of each support portion 2 has increased.
   Fig. 3(c) shows the modification in which each of transition zones T has extended to an optical portion inside an edge portion la. A desired lens thickness can be ensured without impairing the characteristic of each support portion 2 and particularly sacrificing a visual field region of an eye to insert the intraocular lens therein. Incidentally, a region from which a central portion of an optical portion 1, i.e., a 3-mm range is omitted, may preferably be used as a region in which the thickness of the optical portion inside the edge portion la changes. The thickness of each transition zone T on the support portion side is substantially made uniform. In this condition, each transition zone T may be provided on the optical portion side alone so as to fall within a range in which a central portion having a 3-mm range is excluded.
   Figs. 4(a) to 4(b) illustrate modifications of the intraocular lens of the type according to the second embodiment of the present invention. The contents shown in Figs. 4(a) through 4(c) conform to those shown in Figs. 3(a) to 3(c). Fig. 4(a) shows the modification in which each of transition zones T is provided at some midpoint in each support portion 2'. Fig. 4(b) illustrates the modification in which each transition zone T is provided so as to extend from the tip portion of each support portion 2'. Fig. 4(c) depicts the modification in which each transition zone T has extended up to an optical portion inside an edge portion 1a'.
   The intraocular lenses of the present invention have been described above based on the drawings. However, the configurations of the optical portion and the support portions are not necessarily limited to those shown in the drawings. The optical portion may be configured in the form of an ellipse, for example. Even where the optical portion is deformable, i.e., a soft intraocular lens is brought to a so-called single piece type, the present invention can be applied to prevent the thickness of the lens from being excessively thinned due to the low degree thereof.
   In the embodiments described above, the transition zones T have been provided with a view toward ensuring the sufficient thickness of the lens even in the case of the low degree of the lens. However, the transition zones T may also be used to prevent a transmission characteristic from changing due to a change in degree of a so-called colored intraocular lens containing a coloring agent or an ultraviolet-ray absorbing intraocular lens containing an ultraviolet-ray absorbent agent.
   For example, colored intraocular lenses in which coloring agents have been contained with a view toward correcting visual abnormalities such as a cyanopia (this sort of intraocular lens is mentioned in USP5,374,663. In the publication, the intraocular lens is disclosed for correcting the cyanopia by containing at least a sort of coloring agent selected among yellow, brown or orange coloring agents as an essential component into lens material, and by adding an ultraviolet-ray absorbing agent or a briging monomer as an arbitrary component into lens material), may preferably be substantially the same in transmissivity characteristic regardless of the degrees thereof. However, the transmissivity of the lens changes in proportion to its thickness (its transmissivity is determined depending on how much light-absorbing substances exist in an optical path). Therefore, when the degree of the lens is changed not so as to impair the characteristic of each support portion with the thickness of the edge portion as the reference, the lens whose degree is low, is different in lens thickness from the lens whose degree is high and the transmissivity characteristic is also changed. Thus, although the edge portion of the lens varies in thickness according to the degree of the lens when the degree of the lens is changed with the thickness of the center of the lens as the reference, the intraocular lens corresponding to the change in thickness of the lens edge portion can be obtained without impairing the characteristic of each support portion by providing, at the support portions, transition zones T whose each thickness gradually increases toward the lens edge portion. Since the thickness (thickness of central optical portion) of the center of the lens is the same regardless of the lens degree, the transmissivity characteristics can be maintained at substantially the same levels. The transition zones T can be provided at both the periphery of the lens and the support portions or on the periphery of the lens.
   The foregoing description of the preferred embodiment of the invention has been presented for purposes of illustration and description. It is not intended to be exhaustive or to limit the invention to the precise form disclosed, and modifications and variations are possible in light of the above teachings or may be acquired from practice of the invention. The embodiment chosen and described in order to explain the principles of the invention and its practical application to enable one skilled in the art to utilize the invention in various embodiments and with various modifications as are suited to the particular use contemplated. It is intended that the scope of the invention be defined by the claims appended hereto,

## Claims

1. An intraocular lens adapted to be inserted into an eye after a crystalline lens has been removed for replacing a natural lens, the intraocular lens comprising:
an optical portion (1) having a refractive power;
loop shaped support portions (2) supporting the optical portion; and
a colouring agent contained in at least the optical portion (1),
**characterised in that**
the intraocular lens further comprises a transition portion (T) integrally formed with the optical portion (1) and the support portions (2), the transition portion (T) having a thickness gradually increasing toward the optical portion (1) for connecting the optical portion (1) and the support portions (2), and
wherein the optical portion (1) containing the colouring agent has a centre arranged to have the same thickness regardless of the degree of the refractive power of the optical portion.

2. An implantable intraocular lens according to claim 1, wherein the optical portion (1) has a biconvex shape or a planoconvex shape for producing a myopic refractive power.

3. An implantable intraocular lens according to claim 1 or 2, wherein the colouring agent includes an ultraviolet-ray absorbent agent.

## Patentansprüche

1. Intraokulare Linse, die dazu geeignet ist, in ein Auge eingesetzt zu werden, nachdem eine Augenlinse entfernt worden ist, um eine natürliche Linse zu ersetzen, wobei die intraokulare Linse Folgendes umfasst:
- einen optischen Abschnitt (1), der eine Brechkraft aufweist;
- bogenförmige Halteabschnitte (2), die den optischen Abschnitt halten; und
- ein Färbungsagens, das wenigstens in dem optischen Abschnitt (1) enthalten ist,
**dadurch gekennzeichnet, dass**
- die intraokulare Linse ferner einen Übergangsabschnitt (T) umfasst, der einteilig mit dem optischen Abschnitt
(1) und den Halteabschnitten (2) ausgebildet ist, wobei der Übergangsabschnitt (T) eine Dicke aufweist, die in Richtung des optischen Abschnitts (1) allmählich zunimmt, um den optischen Abschnitt (1) und die Halteabschnitte (2) zu verbinden, und
- wobei der optische Abschnitt (1), der das Färbungsagens enthält, ein Zentrum aufweist, das so angepasst ist, dass es, unabhängig von der Höhe der Brechkraft des optischen Abschnitts, die gleiche Dicke hat.

2. Implantierbare intraokulare Linse nach Anspruch 1, wobei der optische Abschnitt (1) eine bikonvexe oder eine plankonvexe Form aufweist, um eine myopische Brechkraft zu erzeugen.

3. Implantierbare intraokulare Linse nach Anspruch 1 oder 2, wobei das Färbungsagens ein Agens enthält, das ultraviolettes Licht absorbiert.

## Revendications

1. Lentille intra-oculaire destinée à être insérée dans un oeil après qu'un cristallin a été retiré, pour le remplacement d'une lentille naturelle, la lentille intra-oculaire comprenant :
une partie optique (1) possédant un pouvoir de réfraction,
des parties (2) de support en forme de boucle qui supportent la partie optique, et
un agent de coloration contenu au moins dans la partie optique (1),
**caractérisée en ce que**
la lentille intra-oculaire comporte en outre une portion de transition (T) formée en une seule pièce avec la partie optique (1) et les parties de support (2), la portion de transition (T) ayant une épaisseur qui augmente progressivement vers la partie optique (1) pour le raccordement de la partie optique (1) aux parties de support (2), et
dans laquelle la partie optique (1) contenant l'agent de coloration a un centre disposé afin qu'il ait la même épaisseur quel que soit le pouvoir de réfraction de la partie optique.

2. Lentille intra-oculaire implantable selon la revendication 1, dans laquelle la partie optique (1) a une forme biconvexe ou une forme plan-convexe pour la production d'un pouvoir de réfraction de myopie.

3. Lentille intra-oculaire implantable selon la revendication 1 ou 2, dans laquelle l'agent de coloration contient un agent absorbant les rayons ultraviolets.
